# EUROPEAN PATENT APPLICATION

(11) **EP 0 522 502 A1**
(43) Date of publication of application: **13.01.1993**
(21) Application number: 92111493.0
(22) Date of filing: 07.07.1992
(51) Int. Cl.: A61K 35/78

(54) **Inhibitors of sucrase activity from tea**

(30) Priority: 08.07.1991 JP 192694/91
(71) Applicant: MITSUI NORIN CO., LTD., Tokyo (JP)
(72) Inventor: Hara, Yukihiko, Shizuoka-ken (JP); Honda, Miwa, Fujieda-shi, Shizuoka-ken (JP)
(74) Representative: Tauchner, Paul, Dr.

(57) **Abstract**

Disclosed is a composition useful in inhibiting the activity of sucrase to control excess intake of sucrose, thereby preventing corpulence. The sucrase activity inhibitor of the present invention contains tea polyphenol(s) as an active ingredient. The inhibitor has an excellent sucrase activity inhibiting activity and is used without harmful side effects to the human body.

## Description

The present invention relates to a sucrase inhibitor which acts specifically on sucrase to thereby inhibit the activity thereof.

In this over-indulgent age we live in, obesity and other related diseases which afflict many adults are serious social problems. Dieting is considered to be an important means of preventing such illnesses.

Sucrase is a digestive enzyme existing in the mucous membrane of the human small intestine and it functions by hydrolyzing sucrose into D-glucose and D-fructose. The inhibition of sucrase has strong implications for use in obesity and diabeties cases where it may be used to combat the effects of overeating, and to counteract the effect of moderate sucrose intake in diabeties. To date, various sucrase activity inhibitors have been developed, but they have not been found to be sufficiently effective and many of them often have the added risk of harmful side effects. Therefore the development of a drug which will inhibit the activity of sucrase and may be used safely without causing any harmful side effects, is highly desirable.

Various research conducted in order to find a natural inhibitor rather than one produced synthetically, resulted in the discovery that substances contained in tea are effective inhibitors of sucrase. On the basis of this finding, the present invention was achieved. The present invention relates to inhibitors of sucrase activity. Such substances inhibiting sucrase activity consisting essentially of tea polyphenols.

### BRIEF DESCRIPTION OF THE DRAWINGS

Fig. 1 shows time-dependent variation of plasma glucose concentration in Example 2.

Fig. 2 shows time-dependent variation of insulin concentration in blood in Example 2.

Fig. 3 shows time-dependent variation of plasma glucose concentration in Example 4, comparing Polyphenon 100 with instant green tea (IGT).

Fig. 4 shows time-dependent variation of plasma glucose concentration in Example 4, comparing Polyphenon 100 with instant black tea (IBT).

Tea polyphenols which are the active ingredients of the sucrase activity inhibitor compositions of the present invention, include tea catechins of the following general formula (1) and theaflavins of the following general formula (2): 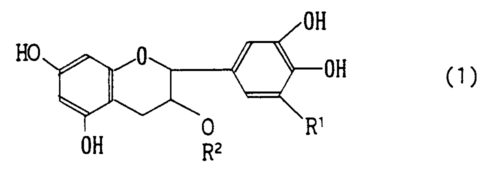
where R¹ represents H or OH; and R² represents H or 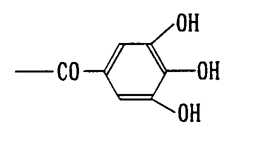
Examples of catechins of the formula (1) are as follows:
(-) Epicatechin (in formula (1), R¹=H, R²=H)
(-) Epigallocatechin (in formula (1), R¹=OH, R²=H)
(-) Epicatechin Gallate (in formula (1), R¹=H, R²= 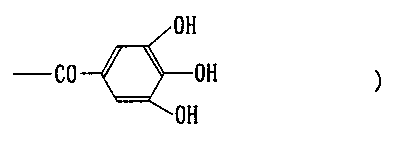
(-) Epigallocatechin Gallate (in formula (1), R¹=OH, R²= 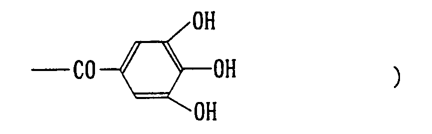 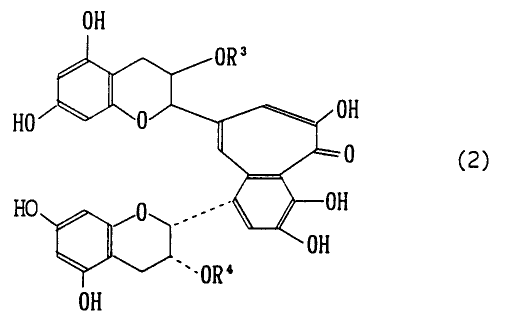
where R³ and R⁴ each represent H or 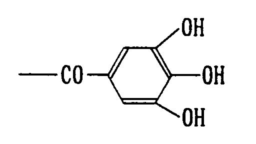
and R³ and R⁴ may be the same as or different from each other.

Examples of theaflavins of formula (2) are as follows: Free Theaflavin (in formula (2), R³=H, R⁴=H)
Theaflavin Monogallate A (in formula (2), R³= 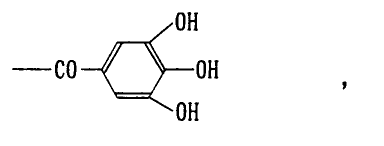
R⁴=H)
Theaflavin Monogallate B (in formula (2), R³=H, R⁴= 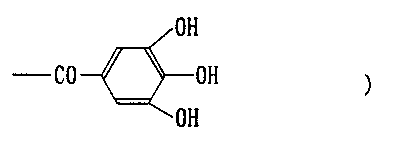
Theaflavin Digallate (in formula (2), R³= 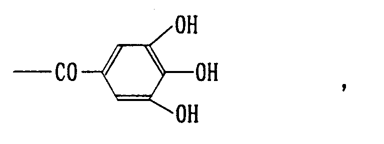
R⁴= 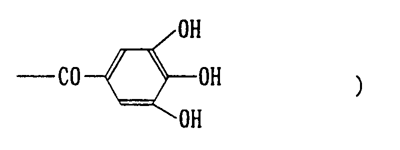
The above-mentioned tea polyphenols can be produced from raw tea leaves, and the method for production is described in U.S. Patent Nos. 4,613,672, 4,673,530 and 4,913,909.

The sucrase activity inhibitor of the present invention can be used in various forms. A suitable amount of the active ingredient, tea polyphenol, may be used singly or it may be dissolved in solvents such as water or alcohols. It may also be blended with a suitable carrier such as gelatin or sodium alginate. If desired, it can be added to food, etc; either directly or after being dissolved in solvents such as water or alcohols.

Suitable amounts of the sucrase activity inhibitor of the present invention to be used in these various ways has been determined. For instance, when it is taken in a medicinal form, from approximately 0.1-10g should be administered perorally, administration, the composition may be such that the active ingredient of the tea polyphenols may be taken directly or it may be blended with a carrier and a diluting agent into powdery, granular or capsule preparations. The intake of tea polyphenols should be such that their concentration in the digestive tubes of the human body is from 0.02mM to 2mM, more preferably from 0.1mM to 1mM.

The sucrase activity inhibitor of the present invention may be used as an additive in food etc; for example, it may be added to various food products such as bread, cereal, noodles and other processed products of rice, potato and corn as well as cakes, biscuits, cookies and confectionery during the manufacturing process, in an amount from 0.01 to 2.0%, or preferably from 0.1 to 1.0%.

The present invention will be explained in more detail by way of the following examples, which, however are not intended to restrict the scope of the invention.

### EXAMPLE 1

Sucrase was prepared from the mucous membrane of the small intestines of Wistar rats in accordance with Dahlquist's Method (A. Dahlquist, Anal. Biochem.,7,18 (1964)).

50 µl of a sample and 100 µl of a substrate solution (substrate: 60mM sucrose solution) were added to 50 µl of an enzyme solution (221 U/ml buffer) and were reacted at 37 °C for 15 minutes. Then the absorbance of the sugar formed by the reaction was measured at 540nm by Bernfeld's method (P. Bernfeld, Meth. Enzymol., 1, 49 (1959)). The value thus measured was converted into the amount of decomposed sucrose, and the reaction speed was calculated using a standard method. The reaction speed of the control case (where distilled water was added instead of the sample solution) was also obtained in the same manner. Sucrase activity in the control case was calculated to be 100%. The percentage inhibition of the samples, each at a final concentration of 5x10⁻⁴ M, were obtained. The results are shown in Table 1.

**Table 1**

| Sample | Inhibition (%) |
|---|---|
| Gallic Acid | 6.7 |
| (+)Catechin | 13.7 |
| Epicatechin | 14.7 |
| Epigallocatechin | 17.3 |
| Epicatechin Gallate | 62.5 |
| Epigallocatechin Gallate | 78.8 |
| Free Theaflavin | 6.8 |
| Theaflavin Monogallate A | 45.9 |
| Theaflavin Monogallate B | 59.5 |
| Theaflavin Digallate | 95.9 |

It was confirmed that out of the catechins and theaflavins tested, epicatechin, epigallocatechin and free theaflavin had only slight sucrase activity inhibiting capacity, but the remaining catechins and theaflavins had strong sucrase activity inhibiting capacity (Table 1).

### EXAMPLE 2

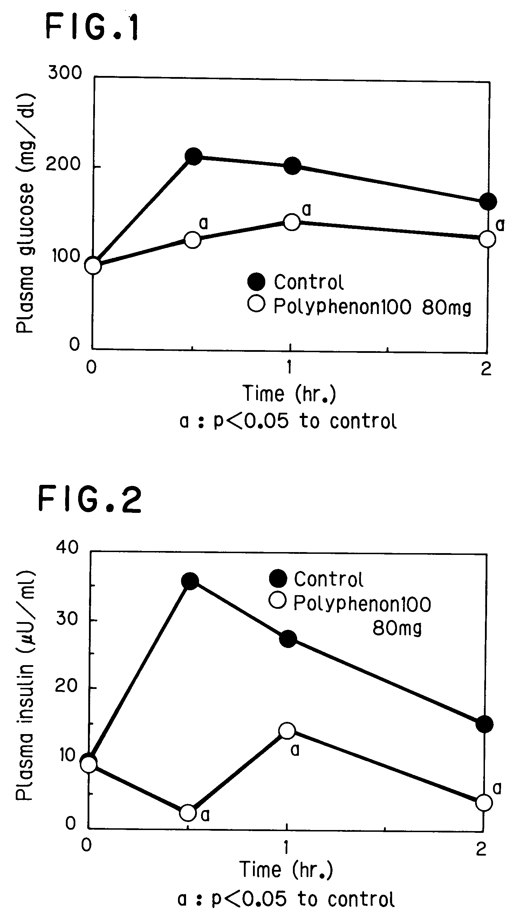

Wistar rats (6 weeks old) were divided into two groups. Rats of one group (control) were fed with water only, while 1ml of an aqueous solution (80 mg/ml) containing crude catechins (Polyphenon 100, product of Mitsui Norin Co. Ltd., the composition of the product is shown in Table 2 below) was perorally administered to each rat of the other group (test group). The plasma glucose level and plasma insulin concentration in the test animals was measured at determined intervals by a mutase/GTO method and a one-step enzyme immunoassay method. The results are shown in Fig. 1 and Fig. 2. As is obvious from these graphs, elevation of the blood glucose level was significantly inhibited when catechins were administered to the test animals before the administration of sucrase. It was also confirmed that the blood insulin concentration decreased. From these facts, it was obvious that the absorbance of glucose decreased due to the sucrase inhibiting activity of catechins and that the blood glucose level and blood insulin concentration was consequently reduced.

**Table 2**

| Composition of Polyphenon 100 | | |
|---|---|---|
| Constitutive Components(tea catechins) | Absolute Propotion(%) | Relative Propotion(%) |
| Gallocatechin | 1.44 | 1.6 |
| Epigallocatechin | 17.57 | 19.3 |
| Catechin | - | - |
| Epicatechin | 5.81 | 6.4 |
| Epigallocatechin Gallate | 59.3 | 59.1 |
| Epicatechin Gallate | 12.51 | 13.7 |

### EXAMPLE 3

An acute toxicity test of the sucrase activity inhibitor of the present invention was carried out using male ICR mice. LD₅₀ was calculated by the Van der Waerden method. The results obtained are shown in Table 3 below.

**Table 3**

| Components | LD₅₀(reliable limit) mg/kg | |
|---|---|---|
| | peroral administration | intraabdominal administration |
| Crude Catechin(*) | 2412 | - |
| Crude Theaflavin(**) | - | 55.2 |
| Epigallocatechin Gallate | - | 150 |

| | | |
|---|---|---|
| (*) Composition of crude catechin:see Table 2 above | | |
| (**) Composition of crude theaflavin:see Table 4 below | | |

**Table 4**

| | |
|---|---|
| Free Theaflavin | 10.0 (%) |
| Theaflavin Monogallate A | 22.3 |
| Theaflavin Monogallate B | 19.5 |
| Theaflavin Digallate | 32.5 |
| (+)Catechin | 0.3 |
| (-)Epicatechin | 1.8 |
| (-)Epigallocatechin Gallate | 4.7 |
| (-)Epigallocatechin Gallate Isomer | 1.0 |
| (-)Epicatechin Gallate | 3.9 |
| Others(Theaflavin Isomers,etc.) | 4.0 |

The tolerance level is usually considered to be 1/3 of the LD₅₀. In this case as compared to the LD₅₀ only a very small amount of the sucrase activity inhibitor (0.1-10g) is necessary to obtain sufficient sucrase activity inhibiting effect. Therefore the sucrase activity inhibitor of the present invention is very safe and it may be used not only for medicinal purposes, but also as an additive to food, without fear of harmful side effects to the human body.

### Example 4

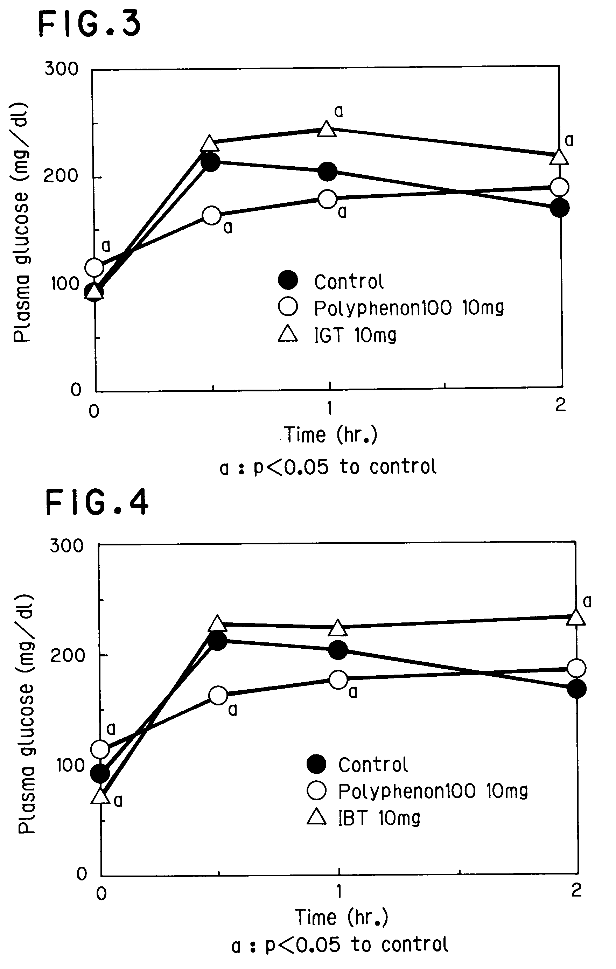

The effect of instant green tea (IGT) and instant black tea (IBT) on the glucose concentration in the blood of rats administered sucrose was studied and no significant differences were found as compared with the control(Figs. 3 and 4). At a concentration of 10mg, corresponding to about 6 cups of tea a day for humans, there were no significant differences in the blood glucose level. From these results it is clear that merely drinking tea, at an average of 6 cups a day, will not reduce the blood glucose level.

Thus, the sucrase activity inhibitor of the present invention is extremely useful for inhibition of sucrase activity in the human body.

## Claims

1. Use of a tea polyphenol selected from the group consisting of epigallocatechin gallate, epicatechin gallate, theaflavin monogallate A, theaflavin monogallate B and theaflavin digallate for the preparation of a pharmaceutical composition useful in inhibiting the activity of the sucrase.

2. Use according to claim 1, wherein said tea polyphenol is epigallocatechin gallate.

3. Use according to claim 1, wherein said tea polyphenol is epicatechin gallate.

4. Use according to claim 1, wherein said tea polyphenol is theaflavin monogallate A.

5. Use according to claim 1, wherein said tea polyphenol is theaflavin monogallate B.

6. Use according to claim 1, wherein said tea polyphenol is theaflavin digallate.

7. Use according to claim 1, wherein the tea polyphenols are present in an amount such that 0.1-10g of tea polyphenols are administered per day.

8. Use according to claim 1, wherein the tea polyphenols are present in an amount such that 0.3-5g of tea polyphenols are administered per day.

9. Use according to any one of claims 1 to 8, wherein the pharmaceutical composition prepared may be used as an food additive.
